# EUROPEAN PATENT APPLICATION

(11) **EP 2 123 257 A2**
(43) Date of publication of application: **25.11.2009**
(21) Application number: 09158415.1
(22) Date of filing: 22.04.2009
(51) Int. Cl.: A61K 9/107, A61K 47/10, A61K 47/14, A61K 47/24

(54) **Stable high Vitamin C content polyol-in-oil emulsified system and its preparation**

(30) Priority: 20.05.2008 CN 200810100726
(71) Applicant: Evonik Goldschmidt GmbH, 45127 Essen (DE)
(72) Inventor: Zhang, Haizhou, Minhang District Shanghai (CN); Dai, Jingya, Minhang District Shanghai 201108 (CN); Zou, Jiali, Minhang District Shanghai (CN); Gao, Zhiheng, Shanghai (CN)

(57) **Abstract**

The present invention is directed to a polyol-in-oil composition used to transport and stabilize Vitamin C, comprising 0.5 wt% to 5 wt% emulsifiers, 0.1 wt% to 5 wt% stabilizer, 0.01 wt% to 20 wt% Vitamin C, 10 wt% to 40 wt% oil components and 40 wt% to 80 wt% polyols, each based on the mass of the whole composition, wherein the proportion of water in the whole composition is less than 1 wt% and wherein Vitamin C is dissolved in the polyols and the use of this composition.

## Description

### Field of the Invention

The present invention relates to a stable high Vitamin C content polyol-in-oil emulsified system and its preparation method. This composition can be widely applied in personal care products field.

### Background

Vitamin C is well known for its various biological functions, such as the stimulation of collagen synthesis, the strengthening of skin tissue against external attack (UV radiation, pollution), depigmentation, anti free radicals. However, due to its alpha-keto lactone structure, Vitamin C is very sensitive to the influence of environmental parameters such as water, light, oxygen, and heavy metals. Degradation of vitamin C in water solution occurs over time, which limits the application vitamin C in cosmetic products.

CN1466939A teaches that Vitamin C stability could be improved by the combination of propylene glycol or glycerin with water. As a mater of fact, US4983382, US5981578, US5736567 and US6020367 also describe this information. Although a certain effect is detectable the product color still deteriorates to yellow. CN1223567A records that the solubility of Vitamin C in non-water media is relatively low and that the active concentration of Vitamin C in such system is also low. The document further mentions that the combination of dimethyl isosorbide, dimetihicone and alcohols can improve the stability of Vitamin C, but this system is not an emulsion, all these components are just mixed, thus it may not be stable with time if used as a cosmetic product. Also the Vitamin C in this system is dispersed as crystals but not dissolved Therefore this system can not be a good Vitamin C transportation system for skin application.

CN1251773A relates to the use of a silicone gum to stabilize ascorbic acid or one of its esters or salts in a topical composition wherein using dimethicone copolyol, ABIL^{®} EM 90, Cyclopentasiloxane and glycerin. But the composition also contains some water.

A lot of references mention that ABIL^{®} EM 90 could be used as the emulsifier of W/O system, wherein the water could includes ethanol or other polyols, e.g. CN1520282A, CN1260166A, CN1199608A, CN1251773A, CN1466939A, CN1592603A, CN1223567A, CN1370062A, CN1223569A, CN1328817A and CN1471903A. And CN1520282A clearly teaches ABIL^{®} EM 90 could be used in a polyols in silicone system.

Hydrophobic silica is always used as a stabilizer in W/O system, e.g. CN1471903A and CN1173125A. CN1471903A relates to a foundation in the form of a W/O emulsion comprising a fatty phase, an aqueous phase, at least one surfactant chosen from C8-C22 alkyl dimethicone copolyols, at least one other surfactant chosen from dimethicone copolyols, and hydrophobic coated pigments. In its description, one of the C8-C22 alkyl dimethicone copolyols could be ABIL^{®} EM 90. The system could contain stabilizer such as AEROSIL^{®} R 812, other hydrophilic components such as propylene glycol and glycerin, and active components such as Vitamin C.

In WO 2006/012723 a polyol/oil-phase composition is described, where the Vitamin C is dissolved in the Polyol phase at elevated temperatures and after cooling down to room temperature the oil phase is added to the Polyol phase. The drawback of this composition is the need of a relatively large amount of emulsifier. Therefore, an optimized system is still expected, which can sufficiently dissolve and stabilize Vitamin C, have a comfortable skin feeling when applied, be stable with shelf time and friendly industrial process.

### Summary of the invention

It was therefore an object of this invention to provide a composition which is capable to dissolve a high amount of Vitamin C without one or more of the disadvantages of the prior art.

Surprisingly we found that an emulsion system comprising the polyol-in-oil composition as defined in the claims by choosing an optimized emulsifier. This emulsion has the traditional white and shiny appearance, and the Vitamin C inside is dissolved in polyols not in water, which lowers the contact of oxygen and metal ions with Vitamin C.

Because the content of oxygen and metal ions in polyols is much lower than that in water, the stability of dissolved Vitamin C can be dramatically increased in this system due to less oxidation. As the system of the invention is or can be essentially free of water, it is not necessary to use preservatives, and therefore it is less harmful to the skin. The optimized emulsifier Cetyl PEG/PPG-10/1 dimethicone (ABIL^{®} EM 90) together with optimized hydrophobic aerosil make the cream made by this system a shinny and white appearance. This cream is very stable with time and can easily be used in personal care field.

Furthermore, the polyols particles (droplets) are encapsulated in the emulsion system by oil components, thus the risk of oxidation is decreased further.

The present invention solves the problem that Vitamin C is quickly degraded in a normal cosmetics product. This invention also provides an innovative emulsified system which is a waterless polyol-in-oil emulsion and can be a stable cosmetic formula.

Although the concept of polyols in oil is proposed in CN1520282A, it is only used for a pre-composition which is part of the final formula. And in this patent, the weight ratio of emulsifiers to polyols is from 100:1 to 2:8. If the content of polyols is close to 50 wt%, the emulsifier quantity would be around 13 wt%, which is obviously too high for a cosmetics formula.

### Detailed description of the invention

The claimed polyol-in-oil composition (polyol-in-oil emulsion system) according to the present invention, useful to transport and stabilize Vitamin C, comprises of from 0.5 wt% to 5 wt% (% by weight), preferably from 1 wt% to 4.5 wt% and more preferably from 2.5 wt% to 3.5 wt% of emulsifiers, from 0.1 wt% to 5 wt%, preferably from 1 wt% to 4 wt% stabilizers, from 0.01 wt% to 20 wt%, preferably from 5 wt% to 15 wt% ascorbic acid (Vitamin C), from 10 wt% to 40 wt% oil components and from 40 wt% to 80 wt%, preferably from 45 wt% to 70 wt% and more preferably from 50 wt% to 65 wt% of polyols, each based on the mass of the whole composition, wherein the proportion of water in the whole composition is less than 1 wt%, preferably less than 0.1 wt%, and wherein Vitamin C is dissolved in the polyols. Preferably the amount of water in the composition is essentially 0 wt%. More preferably no measurable amount of water is contained in the composition of the invention. The optimized content of Vitamin C in the composition is from 10 wt% to 15 wt%.

The emulsifier(s) used in the polyol-in-oil composition of the present invention are preferably selected from cetyl PEG/PPG-10/1 dimethicone (for example ABIL^{®} EM 90 of Evonik Goldschmidt GmbH) and a combination of poly glyceryl alkyl esters. A preferred combination of poly glyceryl alkyl esters is selected from the combination of poly glyceryl alkyl esters containing 1 portion by weight of polyglyceryl-4 diisostearate /polyhydroxystearate /sebacate and 0.25 to 4 portion by weight of polyglyceryl-4 stearate, and from the combination containing 1 portion by weight of diisostearoyl polyglyceryl-3 dimer dilinoleate and 0.25 to 4 portion by weight of polyglyceryl-4 stearate. The molecular weight of the preferred cetyl PEG/PPG-10/1 dimethicone (Evonik Goldschmidt GmbH, ABIL^{®} EM 90) is about 14000g/mol. The structure of preferred cetyl PEG/PPG-10/1 dimethicone is given in formula (**I**), 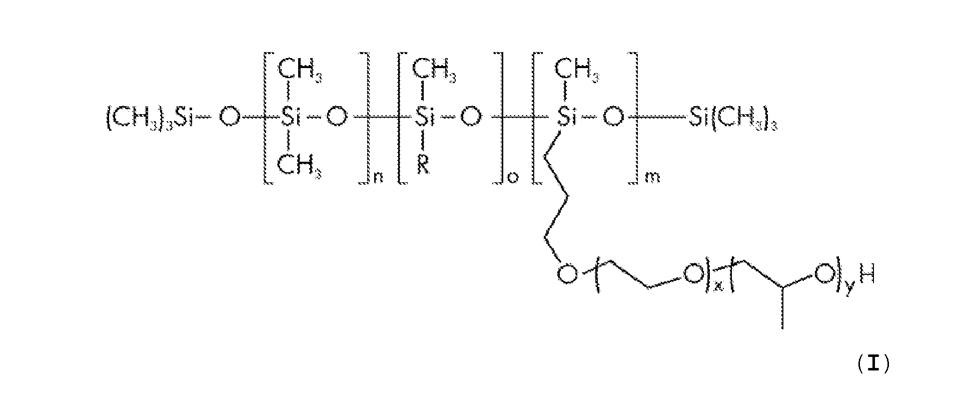 wherein R is a Cetyl group, X=10, Y=1, n=1 to 200, o=1 to 100, m=1 to 40.

Said stabilizers contained in the composition of the present invention are preferably selected from hydrophobic bentonite or hydrophobic silica, especially hydrophobic pyrogenic silica (available under the trade name AEROSIL^{®} from Evonik Degussa GmbH). Preferably the stabilizers are selected from nanometer sized or micrometer sized particles of hydrophobic bentonite or hydrophobic silica. The BET value of a preferred pyrogenic silica is of from 200 to 300m²/g. The methanol wettability of a preferred pyrogenic silica is of from 50 to 70. Preferred stabilizers are AEROSIL^{®} R 812 S from Evonik Degussa GmbH (hydrophobic pyrogenic silica) or bentonite stabilizer a hydrophobic bentonite from Elementis Specialties available under the trade name Bentone^{®} 38V. The above mentioned pyrogenic silica in this invention is preferably made by a pyrolysis method.

The composition according to any of the preceding claims, wherein said stabilizers are chose from nanometer sized or micrometer sized particles of hydrophobic bentonite or hydrophobic silica.

The composition according to claim 6, wherein the BET value of said hydrophobic silica is from 200 m²/g to 300 m²/g and its methanol wettablity is from 50 to 70.

The preferable oil components of the composition of the present comprise silicones, organic modified silicones, fatty hydrocarbons, fatty alcohols and/or fatty esters. Preferred silicones and organic modified silicones are chosen from cyclomethicone (Dow Corning^{®} 345), dimethicone (Goldschmidt, ABIL^{®} 350) or silicone copolymer (Dow Corning^{®} 9040) or a combination thereof. The fatty hydrocarbons, fatty alcohols, fatty esters are preferably selected from paraffin oil, polyisobutene, cetyl alcohol, stearyl alcohol or their mixture, octyldodecanol, cetearyl isononanoate, cetyl ethylhexanoate, cetyl ricinoleate, caprylic/capric triglyceride, decyl oleate, diethylhexyl carbonate, decyl cocoate, isocetyl palmitate, cetearyl ethylhexanoate, isopropyl myristate, myristyl myritate, octyl palmitate, octyl stearate, isopropyl palmitate, triisostearin, steryl heptanoate, 12-15 alkyl benzoate, PPG-15 stearyl ether, PPG-14 butyl ether, PPG-3 myristyl ether or PPG-11 stearyl ether or mixtures thereof.

The polyols used in the present invention preferably are monohydric, dihydric, or trihydric alcohols preferably comprising less than 5 carbons. More preferably the polyol is selected from glycerin, propylene glycol, 1,3-butylene glycol and ethanol or a mixture thereof. Conventional, monohydric alcohols and polyols are totally different materials. But in the present invention, for convenience, monohydric alcohols, especially ethanol, are taken as part of the polyols.

It might be an advantage to add ethanol as a monohydric alcohol to the polyol-in-oil composition of the invention. The amount of mono alcohol, especially of ethanol in the composition of the invention is preferably from 0 wt% or 1 wt% to 10 wt%, more preferably from 2.5 wt% to 5 wt%.

The polyol-in-oil composition (emulsion system) of the present invention might be produced by any method known in the art. Preferably the polyol-in-oil composition (emulsion system) of the present invention is prepared using the following process comprising the steps of:
a.) mixing all oil components, emulsifiers and stabilizers, preferably at room temperature (20 °C - 30 °C), preferably under agitation to obtain a homogeneous phase,
b.) adding Vitamin C to one or more polyols and heating up this mixture up to a temperature above room temperature, preferably to a temperature of 90 °C or more, and more preferably to a temperature of from 90 °C to 100 °C till Vitamin C dissolves completely and a homogeneous mixture is obtained which is then brought down to room temperature, and
c.) adding the polyols phase obtained in step b.) into the oil phase obtained in step a.) preferably slowly and under stirring, more preferably at a stirring speed of from 400 to 600 rpm, and then homogenizing the mixture at higher stirring speed, preferably of from 1000 to 16000 rpm, preferably for several minutes, more preferably of from 15 to 30 minutes, to obtain the polyol-in-oil emulsion with a content of Vitamin C. It might be advantageous when one or more alcohols having a boiling point below the maximum process temperature used in step b.), preferably below 90 °C are added to the polyols mixture after the dissolution of the Vitamin C in the polyol phase in step b.) and before step c.). Preferably the polyol phase is cooled down to a temperature of 45 °C or less before the low boiling alcohol is added. The alcohol added is preferably ethanol. The amounts of ingredients preferably used in the process of the invention can directly be taken from the content of the ingredients of the composition of the invention, especially the preferred embodiments of the composition.

The polyols used at the beginning of step b.) are preferably selected from diols or polyols, more preferably selected from glycerin, propylene glycol and 1,3-butylene glycol or mixtures thereof.

Due to the above mentioned technical method, Vitamin C is successfully dissolved in the polyols phase which is also well encapsulated by oil components in the composition of the present invention. Because the solubility of oxygen in polyols or oil components is much lower than in water, the emulsifying system of the present invention extremely protects Vitamin C from Oxygen, and therefore makes it more stable. Furthermore, the composition of the invention is essentially free of water to avoid the catalyzed oxidation by trace amounts of metal ions (i.e. iron ion, cupric ion) often present in water in traces.

Because the composition of the invention is (essentially) free of water it is not necessary to add any preservatives to the composition. It is therefore less harmful to the skin then those products comprising water and preservatives.

With the use of the preferred emulsifier Cetyl PEG/PPG-10/1 dimethicone (ABIL^{®} EM 90) together with the preferred stabilizer hydrophobic pyrogenic silica a cream can be obtained having a shinny and white appearance. This cream is very stable over time and can easily be used in personal care field. Therefore one more aspect of the invention is a personal care product and especially a cosmetic product comprising a polyol-in-oil composition according to the invention. The personal care and cosmetic product can comprise one or more of the compounds known to be used in personal care and cosmetic products.

The personal care and cosmetic products according to the invention can, e.g., comprise at least one additional component chosen from the group consisting of
emollients
emulsifiers and surfactants
thickeners/viscosity regulators/stabilizers
UV/light screening agents
antioxidants
solids
pearlescent additives
insect repellents
preservatives
fragrances
colorants
biogenic active substances
care additives.

Preferred examples of these components are listed in DE 102005011785 and EP 2000124 which are incorporated by reference.

### Figures

The figures below give illustrate the invention without limiting the invention to the figures.

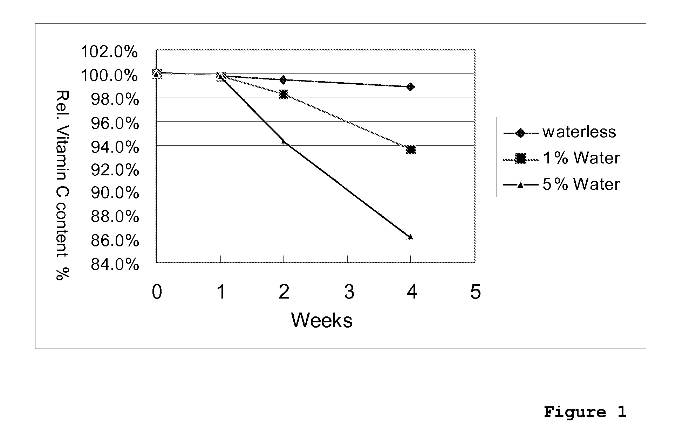

Figure 1, chemical structure of Cetyl PEG/PPG-10/1 dimethicone (ABIL^{®} EM 90)
Figure 1, shows a graph that demonstrates the stability behavior at 50 °C for one month for the compositions of example 1 and comparative examples 1 and 2.

The invention is described in detail by the following examples. The scope of the invention should not be bound to the embodiments of the examples but is given by the scope of the claims.

### Examples

### Example 1 and comparative examples 1 and 2:

The composition of example 1 was prepared in accordance with the process of the invention described above:
a.) 25.4 wt% cyclomethicone (Dow Corning^{®} 345), 5.5 wt% dimethicone (ABIL^{®} 350), 3 wt% emulsifier (ABIL^{®} EM 90), and 3 wt% stabilizer (AEROSIL R 812 S) were mixed at room temperature by stirring to obtain a homogeneous phase.
b.) 10.5 wt% Vitamin C were added into a mixture of polyols of 45 wt% Glycerin and 7.6 wt% propylene glycol. The polyol phase was heated up to about 90 °C and kept there for 30 to 45 minutes till the whole phase was clear and transparent.
c.) The polyols phase was added slowly the into the oil components phase obtained in step a.) at a stirring speed of about 5000 rpm. Then the whole system was mixed at a speed of from 1000 to 16000 rpm till a homogeneous cream was achieved.

The preparation processes for the comparative examples 1 and 2 were performed similar to that of the example 1. The difference was that different amount of water was added after step b.).

**Table 1: Ingredients used to obtain the compositions of example 1 and comparative examples 1 and 2**

| | Ingredients | Example 1 (wt%) | Comparative example 1 (wt%) | Comparative example 2 (wt%) |
|---|---|---|---|---|
| A | Cyclomethicone(Dow Corning^{®} 345) | 25.4 | 25.4 | 25.4 |
| | Hydrophobic silica(AEROSIL R 812 S) | 3 | 3 | 3 |
| | Cetyl PEG/PPG-10/1 dimethicone(ABIL^{®} EM 90) | 3 | 3 | 3 |
| | Dimethicone(ABIL^{®} 350) | 5.5 | 5.5 | 5.5 |
| B | Propylene glycol | 7.6 | 7.6 | 7.6 |
| | Glycerin | 45 | 44 | 40 |
| | Vitamin C | 10.5 | 10.5 | 10.5 |
| | Water | 0 | 1 | 5 |

The content of Vitamin C was analyzed according to the method published in Chinese pharmacopeias year 2000 version, second section, page 791 (ISDN: 7502527362, Chemical Industry Press): After step 3, the content of Vitamin C was analyzed to be 10.3 wt% in example 1. A part of the composition of example 1 was held at room temperature (20 °C - 25 °C) for three months, and then the Vitamin C content was analyzed again. The result showed no Vitamin C degradation. Another part of the composition of example 1 was kept in a completely filled and closed bottle. This bottle was stored in an oven at a temperature of 45 °C for 4 months. The composition was analyzes after the end of this treatment. The Vitamin C content was still 97.1**%** of the original content, which means its degradation is less than 3%. The above mentioned samples were still stable and no separation, roughness and obvious color changing had been observed.

The contents of Vitamin C in comparative examples 1 and 2 were treated and analyzed under the same conditions as example 1. In figure 1 a graph shows the results of the stability measurement. It can easily be seen that the stability of Vitamin C is very sensitive to the water content: a content of 1 wt% of water in the composition of comparative example 1 at 50 °C gives a degradation of 6% after one month and this degradation has already caused an obvious color changing. A content of 5 wt% of water in the composition (comparative example 2) gives a degradation of 14% after one month.

The above test data demonstrates the perfect stability of Vitamin C in the composition of example 1. Furthermore, this composition also passed heat stability test (4 months in 45 °C), cold stability test (one month in - 25 °C) and centrifugation test at 3000 rpm, 30 minutes, which shows its very good stability.

### Example 2:

The preparation process for preparing the composition of example 2 was performed similar to that of the example 1. The difference was that different ingredients (according to table 2) were used to obtain the oil components phase in step a.).

**Table 2: Ingredients used to obtain the compositions of example 2**

| | Ingredients | wt% |
|---|---|---|
| A | Cyclomethicone (Dow Corning^{®} 345) | 16.4 |
| | Silicone copolymer(Dow Corning^{®} 9040) | 10 |
| | Hydrophobic aerosil(AEROSIL R 812 S) | 2 |
| | Cetyl PEG/PPG-10/1 dimethicone(ABIL^{®} EM 90) | 3 |
| | Dimethicone(ABIL^{®} 350) | 5.5 |
| B | Propylene glycol | 7.6 |
| | Glycerin | 45 |
| | Vitamin C | 10.5 |

This composition also passes heat stability test (4 months in 45 °C), cold stability test (one month in - 25 °C) and centrifugation test at 3000 rpm, 30 minutes, which shows its very good stability.

### Examples 3 and 4:

The preparation processes for preparing the compositions of examples 3 and 4 were performed similar to that of the example 1. The difference was that different ingredients (according to table 3) were used to obtain the oil components phase in step a.).

**Table 3: Ingredients used to obtain the compositions of example 3 and 4**

| | Ingredients | Example 3 (wt%) | Example 4 (wt%) |
|---|---|---|---|
| A | Cyclomethicone(Dow Corning^{®} 345) | 3 | 15 |
| | paraffin oil | 15.5 | 15.5 |
| | Hydrophobic aerosil AEROSIL R 812 S) | 1.5 | 1.5 |
| | Polyglyceryl-4 diisostearate/ polyhydroxystearate/ sebacate (Isolan^{®} GPS) | 3 | |
| | Diisostearoyl polyglyceryl-3 dimer dilinoleate (Isolan^{®} PDI) | | 3 |
| | Polyglyceryl-4 isostearate (Isolan^{®} GI 34) | 2 | 2 |
| B | Propylene glycol | | 8 |
| | Glycerin | 60 | 45 |
| | Vitamin C | 15 | 10 |

The emulsifier mixture of Polyglyceryl-4 diisostearate/ polyhydroxystearate/ sebacate (Isolan^{®} GPS) and Polyglyceryl-4 isostearate (Isolan^{®} GI 34) obtained in example 3 showed a good stability. The emulsifier mixuture of Diisostearoyl polyglyceryl-3 dimer dilinoleate (Isolan^{®} PDI) and Polyglyceryl-4 isostearate (Isolan^{®} GI 34) obtained in example 4 showed a shinny and white appearance and was also a stable system.

### Example 5:

The composition of example 5 was prepared in accordance with the process of the invention described above:
a.) 25.4 wt% cyclomethicone (Dow Corning^{®} 345), 5.5 wt% dimethicone (ABIL^{®} 350), 3 wt% emulsifier (ABIL^{®} EM 90), and 3 wt% stabilizer (AEROSIL R 812 S) were mixed at room temperature (20-30 °C) by stirring to obtain a homogeneous phase.
b.) 10.5 wt% Vitamin C were added into a mixture of polyols of 21 wt% glycerin, 7.6 wt% propylene glycol and 20 wt% 1, 3-butylene glycol. This polyol phase was heated up to about 95 °C and kept there for 30 to 45 minutes till the whole phase was clear and transparent. The phase was then cooled down to below 45 °C and then 4 wt% of ethanol were added with agitation.
c.) The polyols phase was added slowly to the oil components phase of step a.) at a speed of about 500 rpm and the whole system was then mixed at a speed of 1000-16000 rpm till a homogeneous cream was achieved.

The ingredients used in example 5 are given in table 4.

**Table 4: Ingredients used to obtain the compositions of example 5**

| | Ingredients | wt% |
|---|---|---|
| A | Cyclomethicone(Dow Corning^{®} 345) | 25.4 |
| | Hydrophobic aerosil AEROSIL R 812 S) | 3 |
| | Cetyl PEG/PPG-10/1 dimethicone(ABIL^{®} EM 90) | 3 |
| | Dimethicone(ABIL^{®} 350) | 5.5 |
| B | Propylene glycol | 7.6 |
| | Glycerin | 21 |
| | 1,3-butylene glycol | 20 |
| | Ethanol | 4 |
| | Vitamin C | 10.5 |

## Claims

1. A polyol-in-oil composition used to transport and stabilize Vitamin C, comprising 0.5 wt% to 5 wt% emulsifiers, 0.1 wt% to 5 wt% stabilizer, 0.01 wt% to 20 wt% Vitamin C, 10 wt% to 40 wt% oil components and 40 wt% to 80 wt% polyols, each based on the mass of the whole composition, wherein the amount of water in the whole composition is less than 1 wt% and wherein the Vitamin C is dissolved in the polyols.

2. The composition according to claim 1, wherein the proportion of water in the whole composition is less than 0.1 wt%.

3. The composition according to claim 1 or 2, wherein the content of Vitamin C in said composition is 5 wt% to 15 wt%.

4. The composition according to any of the preceding claims, wherein said emulsifier is selected from the group containing: Cetyl PEG/PPG-10/1 dimethicone or a combination of poly glyceryl alkyl esters.

5. The composition according to claim 4, wherein the combination of poly glyceryl alkyl esters is chosen from the combination of poly glyceryl alkyl esters containing 1 portion by weight of polyglyceryl-4 diisostearate /polyhydroxystearate /sebacate and 0.25 to 4 portion by weight of polyglyceryl-4 stearate, or 1 portion by weight of diisostearoyl polyglyceryl-3 dimer dilinoleate and 0.25 to 4 portion by weight of polyglyceryl-4 stearate.

6. The composition according to any of the preceding claims, wherein said stabilizers are chose from nanometer sized or micrometer sized particles of hydrophobic bentonite or hydrophobic silica.

7. The composition according to claim 6, wherein the BET value of said hydrophobic silica is from 200 m²/g to 300 m²/g and its methanol wettablity is from 50 to 70.

8. The composition according to any of the preceding claims, wherein said oil components comprise silicones, fatty hydrocarbons, fatty alcohols and/or fatty esters.

9. The composition according to claim 8, wherein the silicones are chosen from cyclomethicone, dimethicone, and silicone copolymer or mixtures thereof, and the fatty hydrocarbons, fatty alcohols and/or fatty esters are chosen from paraffin oil, polyisobutene, cetyl alcohol, stearyl alcohol, octyldodecanol, cetearyl isononanoate, cetyl ethylhexanoate, cetyl ricinoleate, caprylic/capric triglyceride, decyl oleate, diethylhexyl carbonate, decyl cocoate, isocetyl palmitate, cetearyl ethylhexanoate, isopropyl myristate, myristyl myritate, octyl palmitate, octyl stearate, isopropyl palmitate, triisostearin, steryl heptanoate, 12-15 alkyl benzoate, PPG-15 stearyl ether, PPG-14 butyl ether, PPG-3 myristyl ether, PPG-11 stearyl ether or mixtures thereof.

10. The composition according to any of the preceding claims, wherein said polyols are monohydric, dihydric, trihydric alcohols comprising less than 5 carbons.

11. The composition according to claim 8, wherein the polyol is selected from glycerin, propylene glycol, 1,3-butylene glycol and ethanol.

12. A process of producing the polyol-in-oil composition according to any of the preceding claims, which comprises the steps:
a.) mixing all oil components, emulsifiers and stabilizers to obtain a homogeneous phase,
b.) adding Vitamin C to one or more polyols and heating up this mixture up to a temperature above room temperature till Vitamin C dissolves completely and a homogeneous mixture is obtained which is then brought down to room temperature, and
c.) adding the polyols phase obtained in step b.) to the oil phase obtained in step a.) and then homogenizing the mixture to obtain the polyol-in-oil emulsion with a content of Vitamin C.

13. The process of step 12 comprising the steps of:
a.) mixing all oil components, emulsifiers and stabilizers at 20 °C - 30 °C under agitation to obtain a homogeneous phase,
b.) adding Vitamin C into polyols and heating up this mixture up to 90 °C to 100 °C till Vitamin C dissolves and a homogeneous mixture is obtained which is then brought down to room temperature, and
c.) adding the polyols phase obtained in step b.) slowly into the oil phase obtained in step a.) under stirring, and then homogenizing the mixture under higher stirring speed obtaining the polyol-in-oil emulsion with a content of Vitamin C.

14. The process according to claim 12 or 13, wherein after the dissolution of the Vitamin C in the polyol phase in step b.) and before step c.) one or more alcohols having a boiling point below the maximum process temperature of step b.) are added to the polyols mixture.

15. Cosmetic product comprising a polyol-in-oil composition according to one of the claims 1 to 11.
